# EUROPEAN PATENT SPECIFICATION

(11) **EP 2 688 612 B1**
(45) Date of publication and mention of the grant of the patent: **15.07.2015**
(21) Application number: 12710924.7
(22) Date of filing: 22.03.2012
(51) Int. Cl.: A61M 5/24, A61M 5/315, A61M 5/32

(54) **DRUG DELIVERY DEVICE WITH PIVOTING PROTECTIVE CAP**
WIRKSTOFFFREISETZUNGSVORRICHTUNG MIT SCHWENKBARER SCHUTZKAPPE
DISPOSITIF D'ADMINISTRATION DE MÉDICAMENT AVEC CAPUCHON PROTECTEUR PIVOTANT

(30) Priority: 23.03.2011 EP 11159305
(43) Date of publication of application: 29.01.2014
(73) Proprietor: Sanofi-Aventis Deutschland GmbH, 65929 Frankfurt am Main (DE)
(72) Inventor: JUGL, Michael, 65926 Frankfurt am Main (DE); TEUCHER, Axel, 65926 Frankfurt am Main (DE)
(86) International application number: PCT/EP2012/055055
(87) International publication number: WO 2012/126974

(56) References cited:
- EP-A1- 1 346 739
- GB-A- 418 131
- GB-A- 2 461 732
- US-A1- 2008 003 051
- US-A1- 2008 108 951
- US-B1- 7 334 954

## Description

The present invention relates to a drug delivery device adapted to administer a dose of a medicament by way of injection. In particular, the invention refers to pen-type injectors for dispensing a predefined dose of a medicament.

### Background and Prior Art

Drug delivery devices for setting and dispensing multiple doses of a liquid medicament are as such well-known in the art. Generally, such devices have substantially a similar purpose as that of an ordinary syringe.

Drug delivery devices, in particular pen-type injectors have to meet a number of user specific requirements. For instance, with patients' suffering a chronic disease e.g. diabetes, the patient may be physically infirm and may also have impaired vision. Therefore, suitable drug delivery devices need to be robust in construction and should be easy to use. Furthermore, manipulation and general handling of the device and its components should be intelligible and understandable. Moreover, a dose setting and a dose dispensing procedure must be easy to operate and unambiguous. When the device is of disposable type, it should be inexpensive to manufacture and easy to dispose. In order to meet these requirements, the number of parts and steps required to utilize the device should be kept to a minimum.

Typically, the medicament to be administered is provided in a cartridge comprising a moveable piston or bung mechanically interacting with a piston rod of a drive mechanism of the drug delivery device. By applying thrust to the piston in distal direction, a pre-defined amount of the liquid medicament can be expelled from the cartridge.

In particular for elderly or physically infirm users, the overall handling of the device, in particular in a home medication environment should be simple and highly reliable. Typically, drug delivery devices and in particular pen-type injectors comprise a multi-component housing. A distal end section which is adapted to be releasably coupled with a needle assembly is typically protected by a removable protective cap.

For instance; document US 2008/0108951 A1 discloses a storage system for a needleless delivery device syringe and a hypodermic needle adapted for attachment and use with the pen device. There, the storage system comprises a pen needle storage cap that includes a collar which removably snap-fits to a dust sleeve, and which in turn removably snap-fits to the pen delivery device syringe.

Document GB 418 131 A further discloses an opened syringe, having a syringe body and a hinged part, which is pivotally connected with the syringe body by means of a slide.

Document GB 2 461 732 A shows a side loading pen injector comprising a hinged cartridge cover and a plunger. There, the cartridge cover has a pawl engaging with teeth on the outside of a push rod connected to a plunger.

Moreover, document EP 1 346 739 A1 shows a shield hingedly mounted to a distal end of a cartridge holder, which can rotate from an initial configuration into a protected position, where a needle attached to distal end of the cartridge holder is shielded.

In practical use, it may occur, that some of these housing components get lost or immobilize in the course of a dose dispensing or cartridge replacement procedure. For instance, the pen body housing, the cartridge holder and/or the protective cap due to their overall cylindrical geometry may roll away and may drop down from a support structure, e.g. from a table. Hence, In this way, loose and detached components of the drug delivery device may get lost and the end user laboriously would have to look for them.

### Objects of the invention

It is therefore an object of the present invention to provide a drug delivery device being less susceptible to damage and providing an easy and unambiguous handling when in use. It is a further object to encourage or even to oblige the user to make use of protection caps for protecting the distal end section of a cartridge holder, which is typically adapted to receive a piercing assembly. Moreover, it is a particular object of the invention, to prevent damage or loss of the protective cap of a drug delivery device.

### Summary of the Invention

The drug delivery device according to the invention is adapted to dispense a dose of a medicament, e.g. insulin or heparin. The device comprises a housing which is adapted to receive and to house a drive mechanism. The device further comprises a cartridge holder to receive a cartridge containing a medicament. Typically, the cartridge is designed as a carpule, as a vial or as an ampoule and comprises a pierceable seal at a distal end as well as a. moveable piston serving as a proximal seal. By way of a piston rod to be operably engaged with the proximally located piston, distally directed pressure can be applied to the piston in order to expel a pre-defined amount of the medicament via the distally located seal, which is typically pierced or intersected by a piercing element, like an injection needle or cannula. The distally directed forward movement of the piston rod is controlled and conducted by the drive mechanism.

The distal end section of the cartridge holder typically comprises a threaded socket adapted to receive a correspondingly threaded disposable piercing assembly. After use of the device, the piercing assembly is to be detached from the cartridge holder and is intended to be discarded. Since the distal end face of the cartridge holder provides direct access to the penetrable seal of the cartridge it should be protected against environmental influences, in particular against dust and moisture.

In order to protect the cartridge holder and in particular its distally located end section, a protective cap is provided which is adapted to least partially enclose or cover the distal end section of the cartridge holder. The protective cap is pivot mounted on the housing or on the cartridge holder. It cannot be detached therefrom and can therefore not get lost or otherwise damaged during use or maintenance of the drug delivery device.

Generally, the protective cap comprises a cupped geometry. It is pivot mounted on the housing or on the cartridge holder with a proximally located end section. This way, the distally located end section is free to move relative to the cartridge holder. It can simply flapped or turned away in order to provide access to the distal portion of the cartridge holder. When flapped away and transferred into a position of use, the piercing assembly can be mounted on the cartridge holder and a dose setting and dose dispensing procedure may be conducted.

According to a preferred embodiment, the protective cap is firmly connected to the housing or to the cartridge holder. By way of the pivot mount, the protective cap remains permanently attached to the housing or to the cartridge holder of the drug delivery device. It may even be advantageous, when the protective cap is transferable in a position of use, which does not allow to set the drug delivery device aside in a storage container. Hence, for laying away the drug delivery device after use, the user may be obliged to remove and to discard the disposable piercing assembly in order to return the protective cap into its initial position on the cartridge holder. In particular, the size and geometry of the protective cap requires disassembly of the piercing assembly prior to put the protective cap on the cartridge holder.

According to a further preferred embodiment, the pivot axis of the protective cap extends substantially perpendicular to the longitudinal extent of the housing and/or the longitudinal extent of the cartridge holder. Typically, the housing, the cartridge holder and/or the protective cap are of substantially cylindrical geometry. The pivot axis of the protective cap preferably extends in a transverse plane and in radial direction with respect to the cylinder long axis of housing, cartridge holder and/or protective cap. Furthermore, the pivot axis intersects the long axis of the drug delivery device. This way, starting from an initial configuration, the protective cap can be flapped away in a sideward directed motion into a position of use, in which the drug delivery device is ready to set and/or to dispense a dose of the medicament.

For the pivot motion of the protective cap, it is of further benefit, when the cap comprises at least one lateral slit facing towards and/or away from a pivot direction. Typically, the at least one lateral slit corresponds in size with the dimension of the cartridge holder. This way, a sideward directed pivot motion of cartridge holder and protective cap is enabled.

Additionally, the lateral slit may be provided with sealing elements, such like flexible lips or other flexible closure means, like a brush strip allowing for a pivoting displacement of the protective cap relative to the cartridge holder or housing, respectively.

Moreover, it is even of further benefit, when the protective cap comprises two lateral slits arranged on opposite side wall sections of the protective cap. The second lateral slit may face towards the initial pivot direction whereas a second lateral slit may face in opposite direction. In this context, the initial pivot direction denotes the direction the cap is turned to when pivoted from an initial position to a position of use. This way, even a pivot angle of about 180° can be realized allowing to bring the protective cap in overlapping arrangement with the proximally located housing. The first slit is thus adapted to receive the circumference of the housing while the second slit corresponds with the geometry of the cartridge holder.

According to another preferred embodiment, the protective cap and the housing and/or the protective cap and the cartridge holder comprise mutually corresponding fixing means. By way of such fixing means, providing e.g. a positive or frictional interlocking of the protective cap with the housing and/or with the cartridge holder, the cap can be fixed and secured in an initial configuration and/or in a position of use. It is of particular benefit, when the position of use of the protective cap substantially overlaps with the housing of the drug delivery device. Gripping and general handling of the device and its functions can be facilitated in this way.

According to another aspect, the protective cap is slidingly guided on the housing or on the cartridge holder. Starting from an initial configuration, in which the protective cap at least partially covers the cartridge holder, the protective cap may be slidingly displaced initially in distal direction with respect to the cartridge holder in order to enable a pivoting of the protective cap with respect to the cartridge holder. Subsequently, the cap may be pivoted towards the proximal end section of the drug delivery device, hence along the initial pivot direction. In order to receive the proximal end section of the housing, the cap may be slidingly displaced in the opposite, hence, proximal direction and may cover the proximal end of the housing. Subsequently, e.g. for fixing the housing and the protective cap, the cap may be displaced in distal direction along a sliding guide.

According to a further embodiment, the protective cap is slidingly guided in longitudinal direction relative to the cartridge holder and/or relative to the housing, to interlock and/or to release the protective cap and the housing and/or to interlock and/or to release the protective cap and the cartridge holder. A combined sliding and pivoting interconnection of protective cap with either cartridge holder or housing may inherently provide mutual interlocking and releasing of the protective cap with respect to the housing and/or with respect to the cartridge holder.

Finally, the drug delivery device comprises a cartridge being at least partially filled with the medicament to be dispensed. The drug delivery device may be particularly designed as disposable device, wherein an empty cartridge is not intended to be replaced by a filled or new one. Instead, the entire device is intended to be discarded when the medicament contained in the cartridge is used up.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(O2)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or

des Pro36 [Asp28] Exendin-4(1-39),
des Pro36 [IsoAsp28] Exendin-4(1-39),
des Pro36 [Met(0)14, Asp28] EXendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(0)14 Trp(02)25, IsoAsp28] Exendin-4(1-39),
wherein the group -Lys6-NH2 may be bound to the C-terminus of the Exendin-4 derivative;
or an Exendin-4 derivative of the sequence H-(Lys)6-des Pro36 [Asp28] Exendin-4(1-39)-Lys6-NH2, des Asp28 Pro36, Pro37, Pro38Exendin-4(1-39)-NH2, H-(Lys)6-des Pro36, Pro38 [Asp28] Exendin-4(1-39)-NH2, H-Asn-(Glu)5des Pro36, Pro37, Pro38 [Asp28] Exendin-4(1-39)-NH2, des Pro36, Pro37, Pro38 [Asp28] Exendin-4(1-39)-(Lys)6-NH2, H-(Lys)6-des Pro36, Pro37, Pro38 [Asp28] Exendin-4(1-39)-(Lys)6-NH2, H-Asn-(Glu)5-des Pro36, Pro37, Pro38 [Asp28] Exendin-4(1-39)-(Lys)6-NH2, H-(Lys)6-des Pro36 [Trp(02)25, Asp28] Exendin-4(1-39)-Lys6-NH2, H-des Asp28.Pro36, Pro37, Pro38 [Trp(02)25] Exendin-4(1-39)-NH2, H-(Lys)6-des Pro36, Pro37, Pro38 [Trp(02)25, Asp28] Exendin-4(1-39)-NH2, H-Asn-(Glu)5-des Pro36, Pro37, Pro38.[Trp(02)25, Asp28] Exendin-4(1-39)-NH2, des Pro36, Pro37, Pro38 [Trp(02)25, Asp28] Exendin-4(1-39)-(Lys)6-NH2, H-(Lys)6-des Pro36, Pro37, Pro38 [Trp(O2)25, Asp28] Exendin-4(1-39)-(Lys)6-NH2, H-Asn-(Glu)5-des Pro36, Pro37, Pro38 [Trp(02)25, Asp28] Exendin-4(1-39)-(Lys)6-NH2, H-(Lys)6-des Pro36 [Met(O)14, Asp28] Exendin-4(1-39)-Lys6-NH2, des Met(O)14 Asp28 Pro36, Pro37, Pro38 Exendin-4(1-39)-NH2, H-(Lys)6-desPro36, Pro37, Pro38 [Met(O)14, Asp28] Exendin-4(1-39)-NH2, H-Asn-(Glu)5-des Pro36, Pro37, Pro38 [Met(O)14, Asp28] Exendin-4(1-39)-NH2, des Pro36, Pro37, Pro38 [Met(O)14, Asp28] Exendin-4(1-39)-(Lys)6-NH2, H-(Lys)6-des Pro36, Pro37, Pro38 [Met(0)14, Asp28] Exendin-4(1-39)-(Lys)6-NH2, H-Asn-(Glu)5 des Pro36, Pro37, Pro38 [Met(0)14, Asp28] Exendin-4(1-39)-(Lys)6-NH2, H-Lys6-des Pro36 [Met(0)14, Trp(02)25, Asp28] Exendin-4(1-39)-Lys6-NH2, H-des Asp28 Pro36, Pro37, Pro38 [Met(O)14, Trp(02)25] Exendin-4(1-39)-NH2, H-(Lys)6-des Pro36, Pro37, Pro38 [Met(O)14, Asp28] Exendin-4(1-39)-NH2, H-Asn-(Glu)5-des Pro36, Pro37, Pro38 [Met(0)14, Trp(02)25, Asp28] Exendin-4(1-39)-NH2, des Pro36, Pro37, Pro38 [Met(0)14, Trp(02)25, Asp28] Exendin-4(1-39)-(Lys)6-NH2, H-(Lys)6-des Pro36, Pro37, Pro38 [Met(O)14, Trp(02)25, Asp28] Exendin-4(S1-39)-(Lys)6-NH2, H-Asn-(Glu)5-des Pro36, Pro37, Pro38 [Met(O)14; Trip(O2)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, end 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

It will be further apparent to those skilled in the pertinent art that various modifications and variations can be made to the present invention without departing from the scope of the invention. Further, it is to be noted, that any reference signs used in the appended claims are not to be construed as limiting the scope of the present invention.

### Brief Description of the Drawings

In the following, preferred embodiments of the invention will be described in greater detail by making reference to the drawings in which:
- Figure 1: illustrates a pen-type injector in a perspective view according to the prior art,
- Figure 2: shows the pen-type injector according to Figure 1 in an exploded view,
- Figure 3: shows a schematic sketch of a pen-type injector having a pivot-mounted protective cap,
- Figure 4: is illustrative of the device according to Figure 3 with the cap in a first pivot position and
- Figure 5: shows the device according to Figures 3 and 4 with the cap in a second pivot position,
- Figure 6: shows another embodiment of a pivot mounted protective cap hingedly attached to the cartridge holder,
- Figure 7: shows the embodiment according to Figure 6 with the protective cap in a first pivot position, and
- Figure 8: shows the drug delivery device according to Figures 6 and 7 with the protective cap pivoted by about 180°.

### Detailed Description

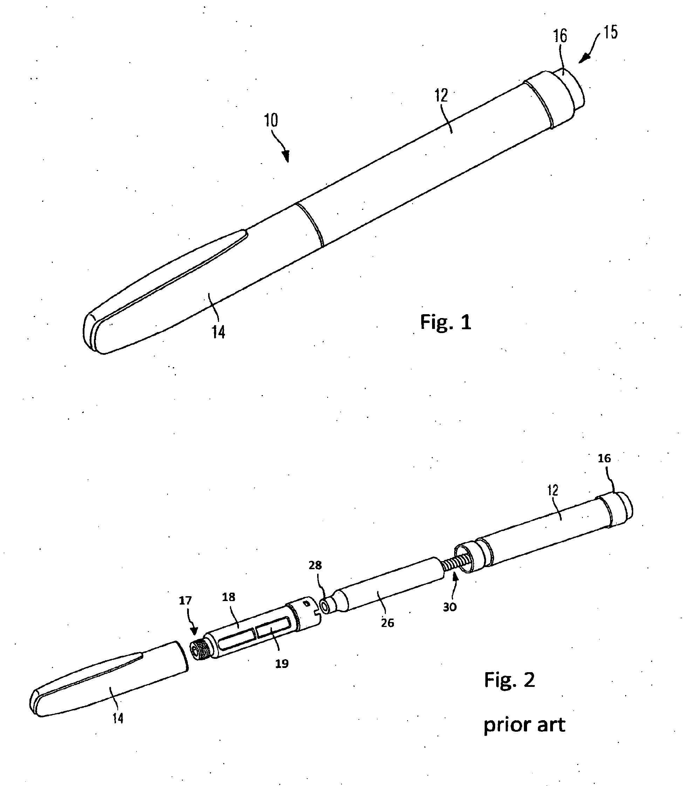

Figure 1 schematically illustrates a drug delivery device 10 according to the prior art designed as a pen-type injector. The device 10 comprises a proximal housing component 12 as well as a distal housing component 14. The distal housing component 14 may be designed as a protective cap, alternatively as a cartridge holder 18 to keep a cartridge 26 filled with a medicament in position. At a proximal end 15, the drug delivery device comprises a dose button 16 by way of which a dose to be dispensed by the device 10 can be individually selected and dispensed. The distal end 17 of the drug delivery device 10 and in particular the distal section of a cartridge holder 18 is adapted to be releasably coupled with a piercing assembly, such like an injection needle, in order to dispense a pre-defined amount of the liquid medicament by way of injection. Therefore, distally located end section 17 of the cartridge holder 18 comprises a threaded socket. The cartridge holder 18 further comprises at least one inspection window 19 in order to visually inspect the filing level of the cartridge 26.

The cartridge 26 itself is at least partially filled with a medicament to be dispensed by the device 10. The cartridge 26 comprises a distally located seal 28 adapted to be intersected or pierced by a piercing assembly, which is not particularly illustrated here. Towards its proximal end section the cartridge 26 comprises a displacabel seal in form of a piston to become operably engaged with the piston rod 30 of a drive mechanism arranged in the proximal housing component 12.

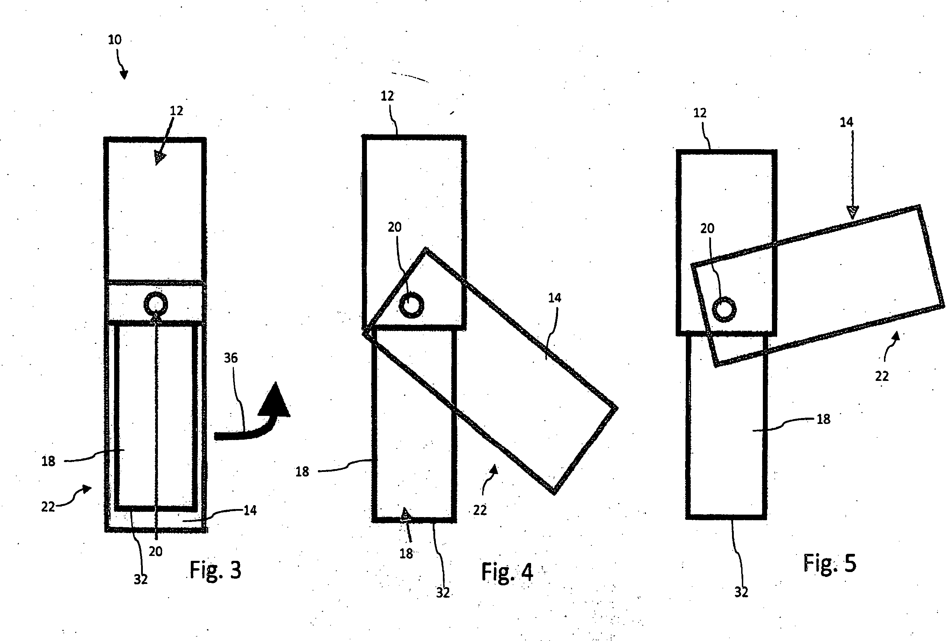

In Figure 3, a simplified view of a drug delivery device 10 according to the present invention is illustrated. The device 10 comprises a proximally located housing component 12 and a protective cap 14 at least partially covering the distally located or distally directed and downward pointing cartridge holder 18. The protective cap 14 is pivot mounted by way of a hinge 20 on a distal portion of the housing 12. As illustrated in the course of Figs. 3 to 5, the protective cap 14 starting from an initial configuration as shown in Figure 3 can be flapped away in a sideward direction 36 in order to provide access to the cartridge holder 18 and its distal end portion 32. The protective cap 14 is connected with the hinge 20 near its proximal portion in order to enable a respective displacement of its distal portion relative to the cartridge holder 18.

In order to provide and to enable a pivoting motion of the protective cap 14, the cap itself comprises at least one lateral slit 22, which, in Figures 3 to 5, is only indicated. Said lateral slit or through opening 22 is arranged on a lateral side wall portion of the protective cap 14 facing away from the initial pivot direction 36 as indicated in Figure 3. This way, the cartridge holder 18 with its distal end section 32 may penetrate said slit or through opening 22 when the protective cap 14 is flapped away. Similarly, when starting from a position of use as indicated in Figure 5, said lateral opening 22 in the protective cap 14 allows to receive the cartridge holder 18 of the drug delivery device 10.

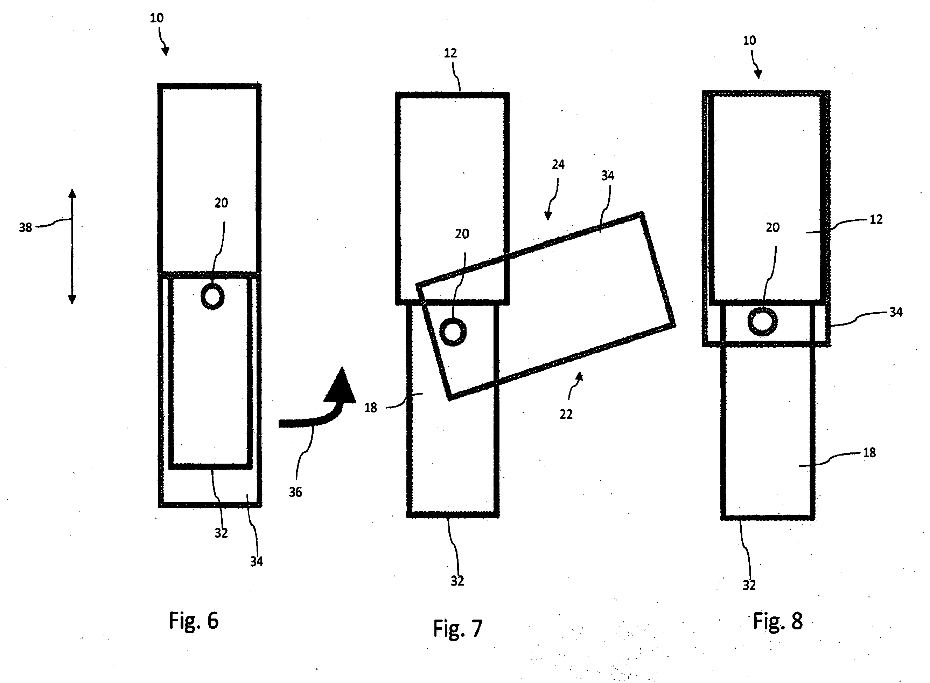

In the embodiment according to the Figs. 6 to 8, the protective cap 34 is pivot mounted on the cartridge holder 18. Furthermore, the protective cap 34 comprises two oppositely located slits or through openings 22, 24. By way of the second through opening 24 facing towards the pivot direction 36, the protective cap 34 can even be pivoted or rotated by about 180°. The cap 34 may therefore receive the proximally located housing 12 of the drug delivery device 10. In such a position of use as indicated in Figure 8, an easy and intuitive handling of the drug delivery device 10 can be provided.

Additionally or according to another embodiment, the protective cap 34 may even be slidingly supported relative to the cartridge holder 18 and/or relative to the housing 12 in distal and/or proximal direction as indicated by the double-tipped arrow 38 in Figure 6. By way of a longitudinal, distally or proximally directed relative displacement of protective cap 34 and cartridge holder 18, mutual interlocking of protective cap 34 and cartridge holder 18 and/or interlocking of protective cap 34 with the housing 12 can be provided. Hence, unintentional pivoting of the protective cap 34 relative to the cartridge holder 18 or relative to the housing 12 can be impeded. Additionally, by providing a translational displacement between cartridge holder 18 and protective cap 34 or between housing 12 and protective cap 34, the size and geometry of the lateral slit or slits 22, 24 can be adapted accordingly.

### List of Reference Numerals

- 10: drug delivery device
- 12: housing
- 14: protective cap
- 15: proximal end
- 16: dose button
- 18: cartridge holder
- 19: inspection window
- 20: hinge
- 22: lateral slit
- 24: lateral slit
- 26: cartridge
- 28: seal
- 30: drive mechanism
- 32: distal end section
- 34: protective cap
- 36: pivot direction
- 38: longitudinal direction

## Claims

1. Drug delivery device for dispensing of a dose of a medicament comprising:
- a housing (12), adapted to receive a drive mechanism (30),
- a cartridge holder (18) to receive a cartridge (26) comprising a medicament,
- a protective cap (14; 34) adapted to at least partially enclose a distal end section (32) of the cartridge holder (18), **characterized by** the protective cap (14; 34) being pivot mounted on the housing (12) or on the cartridge holder (18).

2. The drug delivery device according to claim 1, wherein the protective cap (14;34) is firmly connected to the housing (12) or to the cartridge holder (18).

3. The drug delivery device according to any one of the preceding claims, wherein the pivot axis (20) of the protective cap (14; 34) extends substantially perpendicular to the longitudinal extent of the housing (12) and/or the cartridge holder (18).

4. The drug delivery device according to any one of the preceding claims, wherein the protective cap (14; 34) comprises at least one lateral slit (22, 24) facing towards and/or away from a pivot direction (36).

5. The drug delivery device according to any one of the preceding claims, wherein the protective cap (34) comprises two oppositely arranged lateral slits (22, 24) or lateral through openings.

6. The drug delivery device according to any one of the preceding claims, wherein the protective cap (34) is pivotable by about 180°.

7. The drug delivery device according to any one of the preceding claims, wherein the protective cap (34) and the housing (12) comprise mutually corresponding fixing means.

8. The drug delivery device according to any one of the preceding claims, wherein the protective cap (14; 34) is slidingly guided on the housing (12) or on the cartridge holder (18).

9. The drug delivery device according to claim 8, wherein the protective cap (14; 34) is slidingly guided in longitudinal direction (38) to interlock and/or to release the protective cap (14; 34) and the housing (12) and/or the cartridge holder (18).

10. The drug delivery device according to any one of the preceding claims comprising a cartridge (18) at least partially filled with a medicament.

## Patentansprüche

1. Medikamenten-Verabreichungsvorrichtung zur Abgabe einer Dosis eines Medikaments, umfassend:
- ein Gehäuse (12), das zur Aufnahme eines Antriebsmechanismus (30) ausgelegt ist,
- einen Kartuschenhalter (18) zur Aufnahme einer Kartusche (26), die ein Medikament aufweist,
- eine Schutzkappe (14; 34), die ausgelegt ist, einen distalen Endabschnitt (32) des Kartuschenhalters (18) zumindest teilweise zu umschließen, **dadurch gekennzeichnet, dass** die Schutzkappe (14; 34) schwenkbar an dem Gehäuse (12) oder an dem Kartuschenhalter (18) befestigt ist.

2. Medikamenten-Verabreichungsvorrichtung nach Anspruch 1, wobei die Schutzkappe (14; 34) fest mit dem Gehäuse (12) oder dem Kartuschenhalter (18) verbunden ist.

3. Medikamenten-Verabreichungsvorrichtung nach einem der vorhergehenden Ansprüche, wobei sich die Schwenkachse (20) der Schutzkappe (14; 34) im Wesentlichen senkrecht zur Längserstreckung des Gehäuses (12) und/oder des Kartuschenhalters (18) erstreckt.

4. Medikamenten-Verabreichungsvorrichtung nach einem der vorhergehenden Ansprüche, wobei die Schutzkappe (14; 34) zumindest einen seitlichen Schlitz (22, 24) aufweist, der zu einer Schwenkrichtung (36) hin und/oder von dieser weg weist.

5. Medikamenten-Verabreichungsvorrichtung nach einem der vorhergehenden Ansprüche, wobei die Schutzkappe (34) zwei gegenüberliegend angeordnete seitliche Schlitze (22, 24) oder seitliche Durchgangsöffnungen aufweist.

6. Medikamenten-Verabreichungsvorrichtung nach einem der vorhergehenden Ansprüche, wobei die Schutzkappe (34) um ungefähr 180° schwenkbar ist.

7. Medikamenten-Verabreichungsvorrichtung nach einem der vorhergehenden Ansprüche, wobei die Schutzkappe (34) und das Gehäuse (12) miteinander korrespondierende Befestigungsmittel aufweisen.

8. Medikamenten-Verabreichungsvorrichtung nach einem der vorhergehenden Ansprüche, wobei die Schutzkappe (14; 34) gleitend auf dem Gehäuse (12) oder auf dem Kartuschenhalter (18) geführt ist.

9. Medikamenten-Verabreichungsvorrichtung nach Anspruch 8, wobei die Schutzkappe (14; 34) gleitend in Längsrichtung (38) geführt ist, um die Schutzkappe (14; 34) und das Gehäuse (12) und/oder den Kartuschenhalter (18) miteinander zu verriegeln und/oder voneinander zu lösen.

10. Medikamenten-Verabreichungsvorrichtung nach einem der vorhergehenden Ansprüche, mit einer Kartusche (18), die zumindest teilweise mit einem Medikament gefüllt ist.

## Revendications

1. Dispositif d'administration de médicament destiné à distribuer une dose d'une substance médicamenteuse comprenant :
- un logement (12), conçu pour recevoir un mécanisme d'entraînement (30),
- un support de cartouche (18) destiné à recevoir une cartouche (26) comprenant une substance médicamenteuse,
- un capuchon protecteur (14 ; 34) conçu pour recouvrir au moins partiellement une section d'extrémité distale (32) du support de cartouche (18), **caractérisé en ce que** le capuchon protecteur (14 ; 34) est monté à pivotement sur le logement (12) ou sur le support de cartouche (18).

2. Dispositif d'administration de médicament selon la revendication 1, dans lequel le capuchon protecteur (14 ; 34) est solidement raccordé au logement (12) ou au support de cartouche (18).

3. Dispositif d'administration de médicament selon l'une quelconque des revendications précédentes, dans lequel l'axe de pivotement (20) du capuchon protecteur (14 ; 34) s'étend essentiellement de manière perpendiculaire vis-à-vis de l'étendue longitudinale du logement (12) et/ou du support de cartouche (18).

4. Dispositif d'administration de médicament selon l'une quelconque des revendications précédentes, dans lequel le capuchon protecteur (14 ; 34) comprend au moins une fente latérale (22, 24) orientée dans une direction allant vers et/ou s'éloignant d'une direction de pivotement (36).

5. Dispositif d'administration de médicament selon l'une quelconque des revendications précédentes, dans lequel le capuchon protecteur (34) comprend deux fentes latérales (22, 24) ou deux ouvertures traversantes latérales disposées de façon opposée.

6. Dispositif d'administration de médicament selon l'une quelconque des revendications précédentes, dans lequel le capuchon protecteur (34) peut pivoter d'approximativement 180°.

7. Dispositif d'administration de médicament selon l'une quelconque des revendications précédentes, dans lequel le capuchon protecteur (34) et le logement (12) comprennent des moyens de fixation mutuellement correspondants.

8. Dispositif d'administration de médicament selon l'une quelconque des revendications précédentes, dans lequel le capuchon protecteur (14 ; 34) est guidé en coulissement sur le logement (12) ou sur le support de cartouche (18).

9. Dispositif d'administration de médicament selon la revendication 8, dans lequel le capuchon protecteur (14 ; 34) est guidé en coulissement dans une direction longitudinale (38) afin de verrouiller réciproquement et/ou de libérer le capuchon protecteur (14 ; 34) et le logement (12) et/ou le support de cartouche (18).

10. Dispositif d'administration de médicament selon l'une quelconque des revendications précédentes, comprenant une cartouche (18) au moins partiellement remplie d'une substance médicamenteuse.
